# EUROPÄISCHE PATENTSCHRIFT

(11) **EP 1 600 460 B1**
(45) Veröffentlichungstag und Bekanntmachung des Hinweises auf die Patenterteilung: **15.08.2012**
(21) Anmeldenummer: 04078136.1
(22) Anmeldetag: 11.11.2004
(51) Int. Cl.: C07K 16/28, A61K 38/00

(54) **Verwendung einer an CD28 bindenden Wirksubstanz zur Herstellung einer pharmazeutischen Zusammensetzung zur Behandlung von B-CLL**
Use of an active substance that binds to CD28 for producing a pharmaceutical composition for treatment of B-CLL
Utilisation d'une substance active se liant à CD28 pour la préparation d'une composition pharmaceutique pour traitement de B-CLL

(30) Priorität: 11.11.2003 DE 10352900
(43) Veröffentlichungstag der Anmeldung: 30.11.2005
(73) Patentinhaber: TheraMAB LLC, Moscow 117246 (RU)
(72) Erfinder: Hanke, Thomas, 97076 Würzburg (DE)
(74) Vertreter: Jungblut, Bernhard Jakob

(56) Entgegenhaltungen:
- WO-A-03/048194
- WO-A-03/078468
- THOMAS HANKE: "Binding site for TeGenero's CD28-SuperMAB identified"[Online] 22. Mai 2003 (2003-05-22), XP002346688 Gefunden im Internet: URL:http://www.tegenero.com/documents/pr_j em_paper_22.5.2003.pdf> [gefunden am 2005-09-26]
- ELFLEIN KARIN ET AL: "Rapid recovery from T lymphopenia by CD28 superagonist therapy." BLOOD. 1 SEP 2003, Bd. 102, Nr. 5, 1. September 2003 (2003-09-01), Seiten 1764-1770, XP002346689 ISSN: 0006-4971
- LIN CHIA-HUEY ET AL: "Efficient expansion of regulatory T cells in vitro and in vivo with a CD28 superagonist." EUROPEAN JOURNAL OF IMMUNOLOGY, Bd. 33, Nr. 3, März 2003 (2003-03), Seiten 626-638, XP001156306 ISSN: 0014-2980 (ISSN print)
- LÜHDER FRED ET AL: "Topological requirements and signaling properties of T cell-activating, anti-CD28 antibody superagonists." THE JOURNAL OF EXPERIMENTAL MEDICINE. UNITED STATES 21 APR 2003, Bd. 197, Nr. 8, 21. April 2003 (2003-04-21), Seiten 955-966, XP002261827 ISSN: 0022-1007

## Beschreibung

### Gebiet der Erfindung.

Die Erfindung betrifft die Verwendung einer Wirksubstanz zur Herstellung einer pharmazeutischen Zusammensetzung zur Behandlung der chronisch lymphozytären Leukämie des B-Zell-Typs (B-CLL).

### Hintergrund der Erfindung und Stand der Technik.

Zum Verständnis der Erfindung ist zunächst folgender technologischer Hintergrund wichtig. Die Aktivierung ruhender T-Zellen zur Proliferation und funktionellen Differenzierung erfordert zunächst die Besetzung zweier Oberflächenstrukturen, sogenannter Rezeptoren: 1. des Antigenrezeptors, der von Zelle zu Zelle eine unterschiedliche Spezifität besitzt und für die Erkennung von Antigenen, z.B viralen Spaltprodukten, notwendig ist; sowie 2. des auf allen ruhenden T-Zellen mit Ausnahme einer Untergruppe der CD8 T-Zellen des Menschen gleichermaßen exprimierten CD28 Moleküls, welches natürlicherweise an Liganden auf der Oberfläche anderer Zellen des Immunsystems bindet. Man spricht von der Costimulation der antigenspezifischen Immunreaktion durch CD28. In Zellkultur können diese Vorgänge nachgestellt werden durch Besetzung des Antigenrezeptors sowie des CD28-Moleküls mit geeigneten monoklonalen Antikörpern (mAb). Im klassischen System der Costimulation führt weder die Besetzung des Antigenrezeptors noch die des CD28-Moleküls allein zur T-Zellproliferation, die Besetzung beider Rezeptoren ist jedoch effektiv. Diese Beobachtung wurde an T-Zellen des Menschen, der Maus und der Ratte gemacht.

Dagegen sind auch CD28-spezifische mAbs bekannt, die ohne Costimulation die T-Zellproliferation einleiten können. Eine solche superagonistische, d. h. von der Besetzung des Antigenrezeptors unabhängige Aktivierung ruhender T-Lymphozyten durch CD28-spezifische mAb ist aus der Literaturstelle Tacke et al., Eur. J. Immunol., 1997, 27:239-247 bekannt. Demnach wurden zwei Arten von CD28-spezifischen monoklonalen Antikörpern mit unterschiedlichen funktionellen Eigenschaften beschrieben: costimulatorische mAb, die die Aktivierung ruhender T-Zellen nur bei gleichzeitiger Besetzung des Antigenrezeptors costimulieren; und superagonistische mAb, die ohne Besetzung des Antigenrezeptors T-Lymphozyten aller Klassen in vitro und im Versuchstier zur Proliferation aktivieren können. Aus den Literaturstellen DE 197 22 888, WO 03/048194 A und PCT/DE03/00890 sind ebenfalls CD28 spezifische mAbs bekannt, die T-Lymphozyten sowohl in vitro als auch in vivo ohne TCR Stimulation effizient aktivieren, also "superagonistisch" wirken. Diese mAb, jedenfalls jene, die gegen Human CD28 gerichtet sind, werden auch SuperMAB genannt.

Die chronische lymphatische Leukämie der B-Zellreihe (chronisch lymphozytäre Leukämie des B-Zell-Typs, B-CLL) stellt mit einer Inzidenz von 3/100.000 Einwohner die häufigste Leukämie des Erwachsenenalters dar (Landis et al., 1999). Die Erkrankung ist charakterisiert durch eine progressive Akkumulation maligner monoklonaler B-Lymphozyten in Blut, Lymphknoten, Leber und Knochenmark. Mit Fortschreiten der Erkrankung kommt es zu einer Erhöhung der Lymphozytenzahl im Blut, zu einer Vergrösserung von Lymphknoten, Leber und Milz, sowie zu einer Anämie und Thrombozytopenie (Caligaris-Cappio, 2000; Rozman and Montserrat, 1995). Eine kurative Therapie der B-CLL ist zur Zeit nicht möglich.

Zu den Hauptkomplikationen bei der B-CLL zählen Infektionen, wofür u.a. die bei vielen Patienten beobachteten T-Zelldefekte bzw. niedrige T-Zellzahlen verantwortlich gemacht werden (Cantwell et al., 1997; Scrivener et al., 2003).

Eine eingeschränkte Funktion ist des weiteren auch bei den Tumorzellen selbst zu finden. So ist die Eigenschaft der B-CLL B-Zellen, als antigenpräsentierende Zellen (APZ) zu agieren und somit von tumorspezifischen T-Zellen erkannt und eliminiert zu werden, massiv eingeschränkt. Dies liegt u.a. daran, dass B-CLL B-Zellen nicht oder nur unzureichend die natürlichen CD28 Liganden CD80 (= B7.1) und CD86 (=B7.2) an ihrer Zelloberfläche exprimieren. Somit erhalten die potentiell tumorspezifischen T-Zellen nur ein einziges Signal über die Interaktion zwischen dem T-Zell-Rezeptor und MHC + Tumorantigen und nicht das für die volle Aktivierung einer T-Zelle nötige zweite kostimulatorische Signal. Zusammengefasst weist dies darauf hin, dass es dem Immunsystem von B-CLL-Patienten offensichtlich nicht gelingt, die Tumorzellen aus eigener Kraft zu eliminieren.

Ein Ansatz für eine Therapieform zielt darauf ab, die APZ-Funktion der B-CLL B-Zellen zu verbessern und damit die anti-Tumor Immunantwort des Patienten zu verstärken. Verschiedene Arbeitsgruppen versuchten hierzu, B-CLL B-Zellen gezielt zu aktivieren und damit die Expression kostimulatorischer Liganden zu induzieren. Dabei gelang es, mittels Gentransfer den B-Zell-stimulierenden CD40-Liganden (CD40L) in den B-CLL B-Zellen zu exprimieren und damit die Interaktion zwischen CD40L und dem auf allen B-Zellen (inklusive der B-CLL B-Zellen) vorhandenen Protein CD40 zu ermöglichen. Das über CD40 in das Zellinnere weitergeleitete Signal führte zur gewünschten Expression der kostimulatorischen Liganden CD80 und CD86 (Kato et al., 1998; Wendtner et al., 2002). Die so modifizierten Leukämiezellen konnten nun tatsächlich effektiver als APZ fungieren und wurden nun auch - zunächst *in vitro -* von autologen T-Zellen eliminiert (Kato et al., 1998). Auch der *in vivo* Gentransfer von CD4OL war effektiv: Er führte in den Patienten sowohl zu einer Erhöhung der T-Zellzahl als auch zu einer Reduktion der leukämischen Zellen (Wierda et al., 2000). Dieser therapeutische Ansatz berührt jedoch nicht die vorstehend angesprochenen T-Zell Defekte der B-CLL Erkrankung.

Folgend ist die Bibliographie der vorstehend angesprochenen wissenschaftlichen Literatur angegeben. Caligaris-Cappio, F., Rev Clin Exp Hematol 4, 5-21 (2000). Cantwell, M. et al., Nat Med 3, 984-989 (1997).
Kato, K. et al., J Clin Invest 101, 1133-1141 (1998). Landis, S. H. et al., CA Cancer J Clin 49, 8-31 (1999). Rozman, C. et al., N Engl J Med 333, 1052-1057 (1995). Scrivener, S. et al., Leuk Lymphoma 44, 383-389 (2003). Wendtner, C. M. et al., Blood 100, 1655-1661 (2002). Wierda, W. G. et al., Blood 96, 2917-2924 (2000).

### Technisches Problem der Erfindung.

Der Erfindung liegt das technische Problem zu Grunde, eine pharmazeutische Zusammensetzung anzugeben, mittels welcher B-CLL behandelbar ist, wobei zugleich das krankeitsbedingt geschwächte Immunsystem, insbesondere die T-Zell-vermittelte zelluläre Immunität, allgemein gestärkt wird.

### Grundzüge der Erfindung und bevorzugte Ausführungsformen.

Zur Lösung dieses technischen Problems lehrt die Erfindung den fegenstand des Anspruchs 1.

Die Erfindung beruht auf der Erkenntnis, dass mit dem Einsatz der erfindungsgemäßen mAb gleichsam ein Doppelschlag geführt wird. Zum einen werden normale T-Zellen, welche krankheitsbedingt zurückgedrängt sind, aktiviert bzw. expandiert. Dadurch können mitunter lebensgefährliche Infektionen verhindert oder im Krankheitsverlauf abgeschwächt werden. Gleichzeitig wird das körpereigene Immunsystem angeregt, die normalerweise nicht oder nur schwach von tumorspezifischen T-Zellen angegriffenen B-CLL B-Zellen zu eliminieren, was dadurch gelingt, dass die B-CLL B-Zellen zur Präsentation eines von ihnen normalerweise nicht exprimierten kostimulatorischen Liganden aktiviert werden, wodurch die auf ein solches Signal angewiesenen körpereigenen tumorspezifischen T-Zellen die B-CLL B-Zellen nunmehr effektiv angreifen können.

Ein erfindungsgemäßer mAb ist beispielsweise herstellbar, indem ein nichtmenschliches Säugetier mit CD28 oder einer Teilsequenz hieraus, beispielsweise dem C'-D Loop, immunisiert wird, wobei aus dem nichtmenschlichen Säugetier Zellen entnommen und aus den Zellen Hybridomzellen hergestellt werden, und wobei die so erhaltenen Hybridomzellen mit der Maßgabe selektiert werden, daß in deren Kulturüberstand mAb enthalten sind, die an CD28 superagonistisch binden. Aber auch andere übliche Methoden des Erhaltes entsprechender mAb, wie beispielsweise Phage Display oder Human-Ig transgene Mäuse, sind einsetzbar. Geeignete mAb lassen sich auch dadurch herstellen, dass B-Lymphozyten selektiert werden, welche an CD28 bzw. dessen C'-D Loop superagonistisch binden, und deren exprimierte Immunglobulingene kloniert werden. Für ICOS, CTLA-4 UND PD1 gilt Entsprechendes.

Eine erfindungsgemäße eingesertzer mAb bindet an CD28 oder an eine Teilsequenz hieraus. Die CD28 Teilsequenz kann beispielsweise eine Aminosäurensequenz Seq.-ID 1, oder 2 - 7, oder 17 enthalten, welche zumindest teilweise im Bereich des C'-D Loops von CD28 liegen. An eine der Sequenzen mit Val am 5'-Ende kann eine oder mehrere Aminosäuren der Sequenz 8 in der dort definierten Reihenfolge angeschlossen sein. Das Loop liegt im Bereich mit der Sequenz GNYSQQLQVYSKTGF. Die Bindung kann aber auch an andere Teilsequenzen der Region des C'-D Loops erfolgen, wozu ergänzend auf die Definition der Region des C'-D Loops verwiesen wird.

Der mAb ist beispielsweise erhältlich aus Hybridomzellen, wie hinterlegt unter den DSM Nummern DSM ACC2531 (mAb: 9D7 bzw. 9D7G3H11) oder DSM ACC2530 (mAb: 5.11A bzw. 5.11A1C2H3). Der mAb enthält vorzugsweise eine oder mehrere der Sequenzen Seq.-ID 9, 11, 13 und/oder 15, oder eine oder mehrere Sequenzen Seq.-ID 10, 12, 14 und/oder 16, oder eine oder mehrere Sequenzen 18 und/oder 19, oder hierzu homologe Sequenzen.

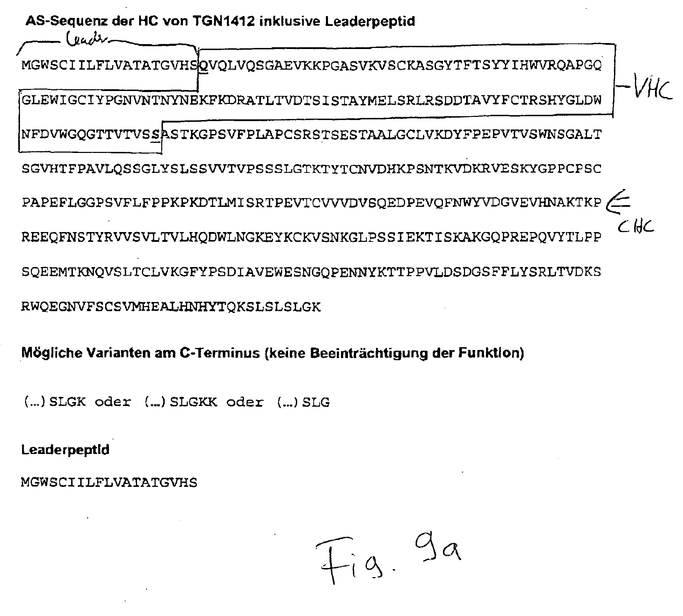

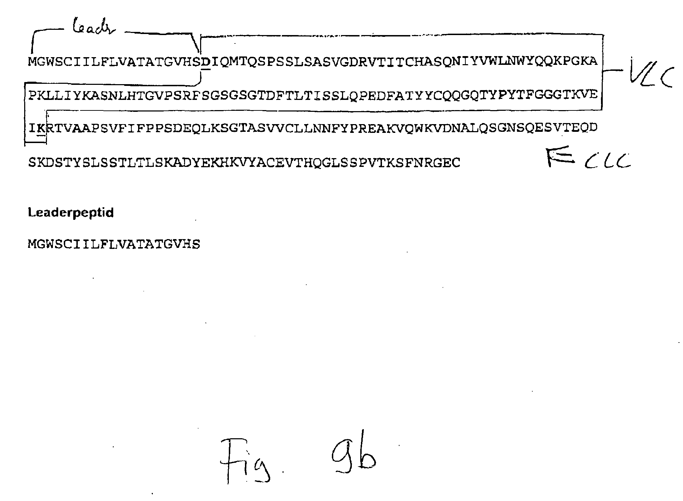

Ein weiterhin im Rahmen der Erfindung geeigneter mAb TGN1412 mit verbesserten Eigenschaften enthält als variable Region der schweren Kette (VHC) die Aminosäuresequenz der Figur 9a und als variable Region der schweren Kette die Aminosäurensequenz der Figur 9b. Die in den Figuren 9a und 9b angegebenen Leaderpeptide sind für die Funktion des mAb irrelevant und dienen lediglich der Erleichterung des Durchtritts der Antikörper durch die Zellmembran. Die in den Figuren angegebenen Varianten des C-Terminus führen zu keiner Veränderung der Funktion der Antikörper.

Die Erfindung ist geeignet für ein Verfahren zur Behandlung von insbesondere B-CLL, wobei entweder einem Patienten eine pharmazeutische Zusammensetzung enthaltend einen erfindungsgemäß eingesetzten superagonistischen monoklonalen Antikörper, galenisch hergerichtet für eine definierte und angewandte Darreichungsform, beispielsweise i.v. Injektion, verabreicht wird, oder einem Patienten eine Körperflüssigkeit (=Zellsuspension), insbesondere Blut, enthaltend T-Lymphozyten und/oder Vorläuferzellen hierzu entnommen wird, die Körperflüssigkeit, ggf. nach einer Aufbereitungsverfahrensstufe, mit einem erfindungsgemäß eingesetzten superagonistischen monoklonalen Antikörper oder einer Mimikriverbindung hierzu versetzt wird und die so behandelte Körperflüssigkeit dem Patienten wieder dargereicht, beispielsweise i.v. injiziert, wird.

Es kann sich empfehlen, wenn vor der Durchführung der Behandlung einer an B-CLL erkrankten Person Blut entnommen wird, darin enthaltene PBMC in-vitro kultiviert werden, die Kultur mit erfindungsgemäß eingesetzten mAb inkubiert wird, und die Aktivierung von T-Zellen und/oder die Eliminierung bzw. Reduktion von B-CLL B-Zellen bestimmt wird. Bezüglich möglicher Bestimmungsmethode wird beispielhaft auf die Ausführungsbeispiele verwiesen, selbstverständlich sind auch andere fachüblich Bestimmungsmethoden und die dafür verwendeten Mittel brauchbar. Dies ist durch führbar mit einem Testkit zur Prüfung auf therapeutische Wirksamkeit einer prospektiven Behandlung von B-CLL mit einer erfindungsgemäßen pharmazeutischen Zusammensetzung mit den folgenden Komponenten: i) Mittel zur in-vitro PBMC Kultivierung, ii) eine Zubereitung enthaltend erfindungsgemäße mAb in physiologisch wirksamer Dosis, iii) Mittel zur Bestimmung der T-Zell Zahlen bzw. Aktivierung und/oder Mittel zur Bestimmung der B-CLL B-Zell Zahlen.

Die dargereichte Tagesdosis für die Indikation B-CLL kann im Bereich von 0,1 bis 50, vorzugsweise 1 bis 10, mg/kg Körpergewicht liegen.

Die Erfindung betrifft schließlich ein Verfahren gemäß Anspruch 5.

### Definitionen.

Als monoklonale Antikörper (mAb) sind Antikörper bezeichnet, die von Hybrid-Zellinien (sog. Hybridomen) produziert werden, die typischerweise durch Fusion einer Antikörper produzierenden B-Zelle tierischer oder menschlicher Herkunft mit einer geeigneten Myelom Tumorzelle entstanden sind.

Die Aminosäuresequenz von Human CD28 ist unter der Accession No. NM_006139 bekannt.

Das C'-D Loop von CD28 umfaßt die Aminosäuren 43 bis 70, insbesondere 52 bis 66, der vorstehenden CD28 Sequenz (zur Numerierung siehe auch Ostrov, D.A., et al.; Science (2000), 290:816-819). Unter dem Begriff des C'-D Loops sollen im Folgenden auch beliebige Teilsequenzen hieraus verstanden sein. Der Begriff der Region des C'-D Loops umfasst zusätzlich oder anstelle des C'-D Loops räumlich benachbarte Bereiche des CD28. Dies sind gemäß der vorstehend genannten Numerierung insbesondere die Aminosäuren 78 (Tyr) bis 87 (Thr).

Ein Loop bzw. eine darin angeordnete Bindungsstelle ist frei zugänglich, wenn für einen definierten Bindungspartner für die Bindungsstelle im Loop keine sterische Hinderung durch an das Loop anschließende Sequenzen oder Moleküle vorliegt.

Mit Aktivierung und/oder Expansion von T-Zellen ist die Vermehrung der Stoffwechselaktivität, Vergrößerung des Zellvolumens, Synthese immunologisch wichtiger Moleküle, wie CD40 Ligand, und Eintritt in die Zellteilung (Proliferation) auf einen äußeren Reiz hin bezeichnet. Im Ergebnis liegen nach der Aktivierung bzw. Expansion mehr T-Zellen vor als vorher.

Homologie bezeichnet eine zumindest 70%ige, vorzugsweise zumindest 80%ige, höchstvorzugsweise zumindest 90%ige Sequenzidentität auf Proteinebene, wobei ein homologes Protein oder Peptid einen definierten Bindungspartner mit zumindest gleicher Affinität bindet. Abweichungen in der Sequenz können Deletionen, Substitutionen, Insertionen und Elongationen sein.

Der Begriff der mAb umfaßt neben Strukturen des üblichen Fab/Fc Aufbaus auch Strukturen, die ausschließlich aus dem Fab-Fragment bestehen. Es ist auch möglich, lediglich die variable Region zu nutzen, wobei das Fragment der schweren Ketten mit dem Fragment der leichten Kette auf geeignete Weise, beispielsweise auch mittels synthetischer Brückenmoleküle, verbunden ist. Der Begriff der Antikörper umfaßt auch (ggf. vollständige) chimäre sowie gentechnologisch humanisierte Antikörper.

Superagonistische Stimulation der Proliferation von CD28 spezifischen T-Zellen meint, daß keine Costimulation, i.e. kein weiteres Bindungsereignis neben einer Bindung eines mAb oder einer Mimikriverbindung an CD28 zur Stimulation oder Inhibierung der Proliferation erforderlich ist.

Im Folgenden wird die Erfindung anhand von lediglich Ausführungsformen darstellenden Beispielen näher erläutert.

### Beispiel 1: SuperMAB induziert die in vitro Expansion von B-CLL T-Zellen

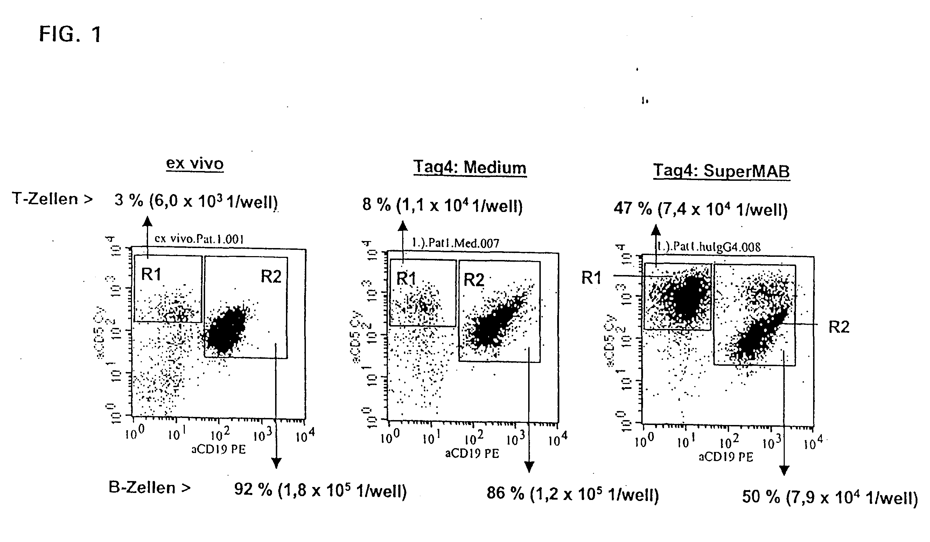

In einem ersten Experiment wurden periphere mononukleäre Zellen aus dem Blut (PBMC) eines B-CLL Patienten isoliert und jeweils 2x10^5 Zellen in 96 Napf Mikrotiterplatten bei 37°C entweder nur in Medium oder in Anwesenheit von SuperMAB (0,5 µg/ml in Lösung) kultiviert. Nach 4 Tagen wurde die Gesamtzellzahl in der Neubauer-Zählkammer bestimmt. Zudem wurden die Zellen mit fluoreszenzmarkierten monoklonalen Antikörpern mit Spezifität für CD19 (B-Zell-Marker) und CD5 (exprimiert auf B-CLL B-Zellen und T-Zellen) inkubiert und im FACS analysiert. Figur 1 zeigt eine sogenannte Dot-Blot Analyse. Jeder Dot stellt eine analysierte Zelle dar und ist entsprechend der Expression von CD19 (x-Achse) und CD5 (y-Achse) lokalisiert. R1 markiert die Population der T-Zellen (CD19-CD5+), R2 die Population der B-CLL B-Zellen (CD19+CD5+). Figur 1 zeigt, dass SuperMAB in der Lage ist, eine massive Expansion der B-CLL T-Zellen zu induzieren. Während in den Ansätzen "ex vivo" und "Medium" die T-Zellen nur 3% bzw. 8% der Gesamtpopulation repräsentieren, stieg dieser Anteil in den SuperMAB behandelten Zellen auf 47% an. Die Analyse der Gesamtzellzahlen zeigte, dass es sich hierbei nicht nur um eine relative sondern auch um eine absolute Vermehrung von T-Zellen handelt. Ausgehend von 6 x 10^3 T-Zellen/Napf (ex vivo) bzw. 1,1 x 10^4 T-Zellen /Napf (Medium) führte die Zugabe von SuperMAB in die Kultur zu einem Anstieg auf 7,4 x 10^4 T-Zellen/Napf. In den SuperMAB-Kulturen konnten nicht nur eine Proliferation der Tumorzellen vermieden, sondern sogar eine Reduktion dieser Zellen um ca. 50% erreicht werden.

In weiteren Experimenten wurde nun untersucht, ob die durch SuperMAB induzierte Expansion der T-Zellen auch mit qualitativen Veränderungen einhergeht und letztendlich auch die Immunantwort in B-CLL Patienten verstärken kann.

### Beispiel 2: SuperMAB induziert die Expression des B-Zell-stimulierenden CD40 Liganden auf der Oberfläche von B-CLL T-Zellen

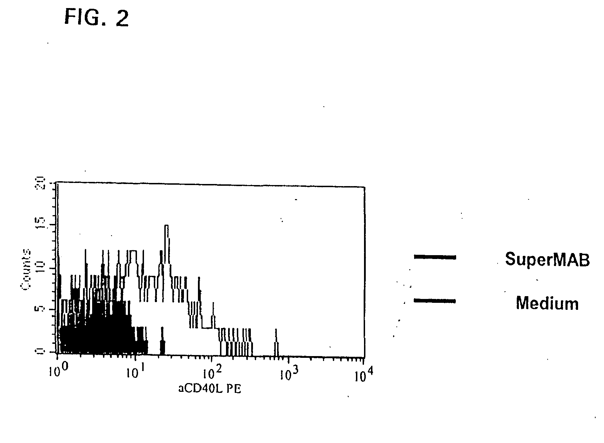

Es konnte bereits in früheren Experimenten gezeigt werden, dass SuperMAB in der Lage ist, die Expression des B-Zell-stimulierenden CD40 Liganden zu induzieren. Es interessierte nun, ob dies auch bei T-Zellen von B-CLL Patienten möglich ist und somit über eine CD40L-CD40 Interaktion B-CLL B-Zellen aktiviert werden können (siehe oben). Wie oben beschrieben wurden PBMC eines B-CLL Patienten in Anoder Abwesenheit von SuperMAB kultiviert und nach einem Tag mit einem fluoreszenzmarkierten Antikörper mit Spezifität für CD40L gefärbt. Figur 2 zeigt ein sogenanntes Histogramm, in dem die Expression von CD40L auf den T-Zellen (x-Achse: da es sich bei PBMC um eine gemischte Kultur handelt, wurden die T-Zellen zuvor durch ein sogenanntes "elektronisches gate" eingegrenzt) gegen die Zellzahl (y-Achse) aufgetragen ist. Im Vergleich zu Zellen, die nur in Medium kultiviert wurden, zeigen die T-Zellen aus der SuperMAB-behandelten Kultur eine deutlich stärkere Expression von CD40L. Somit sind grundsätzlich die Voraussetzungen dafür geschaffen, dass die jetzt CD40L exprimierenden T-Zellen mit dem CD40 auf den B-CLL B-Zellen interagieren und damit die Expression kostimulierender Liganden auf diesen Zellen induzieren können.

### Beispiel 3: SuperMAB induziert die Expression des kostimulierenden Liganden B7.2 auf B-CLL B-Zellen

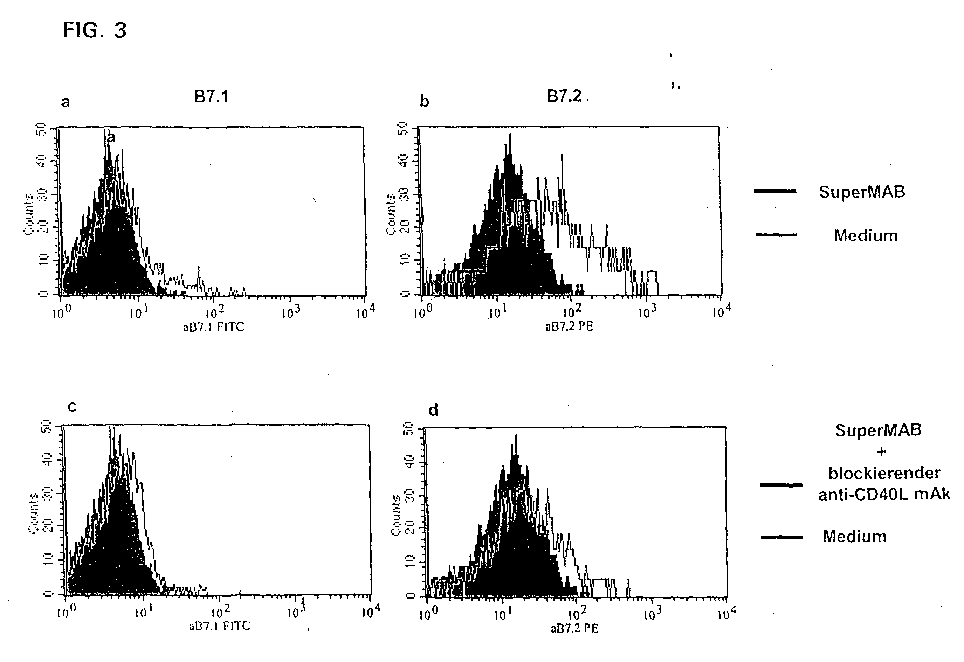

PBMC eines B-CLL Patienten wurden wie oben beschrieben kultiviert und nach 4 Tagen mit fluoreszenzmarkierten monoklonalen Antikörpern mit Spezifität für B7.1 und B7.2 gefärbt. Die Histogramme in Figur 3 zeigen die Expression dieser beiden Proteine auf den B-CLL B-Zellen: Während SuperMAB B7.1 auf diesen Zellen nur mässig induzierte (Histogram a), so konnte er die Expression von B7.2 drastisch steigern (Histogram b). Um beweisen zu können, dass die Induktion von B7.2 die Folge der in Figur 2 gezeigten Expression von CD40L auf SuperMAB behandelten T-Zellen ist, wurde in einem zusätzlichen Ansatz ein blockierender Antikörper (10 µg/ml) mit Spezifität für CD40L in die Kulturen gegeben. Als Folge seiner Fähigkeit, die Interaktion zwischen CD40L und CD40 zu blockieren, sollte somit auch die Induktion von B7.2 auf B-CLL B-Zellen verhindert werden. Die drastisch reduzierte Expression von B7.2 in den so behandelten Zellen (Histogram d) zeigt tatsächlich, dass die Induktion von B7.2 auf B-CLL B-Zellen durch die Blockade von CD4OL verhindert wird. Dieses Ergebnis liefert somit den indirekten Beweis, dass durch SuperMAB aktivierte, CD40L exprimierende T-Zellen mit CD40 auf der Oberfläche von B-CLL B-Zellen interagieren und damit die Expression kostimulierender Proteine induzieren können.

### Beispiel 4: SuperMAB verbessert antigenpräsentierende Funktion von B-CLL B-Zellen (allogene gemischte Lymphozytenkultur)

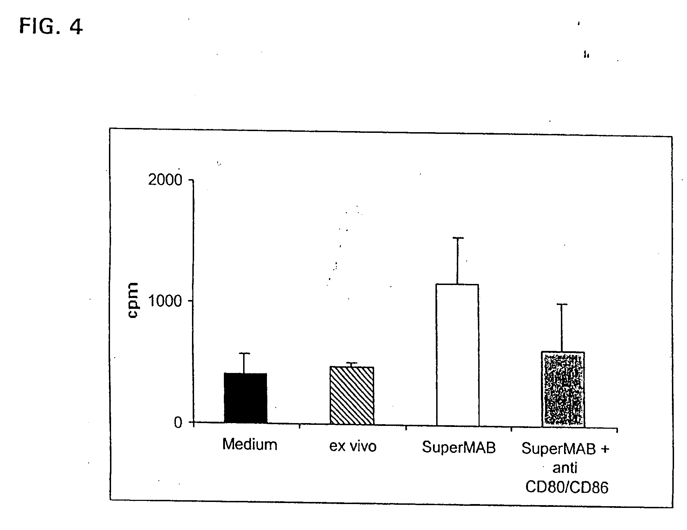

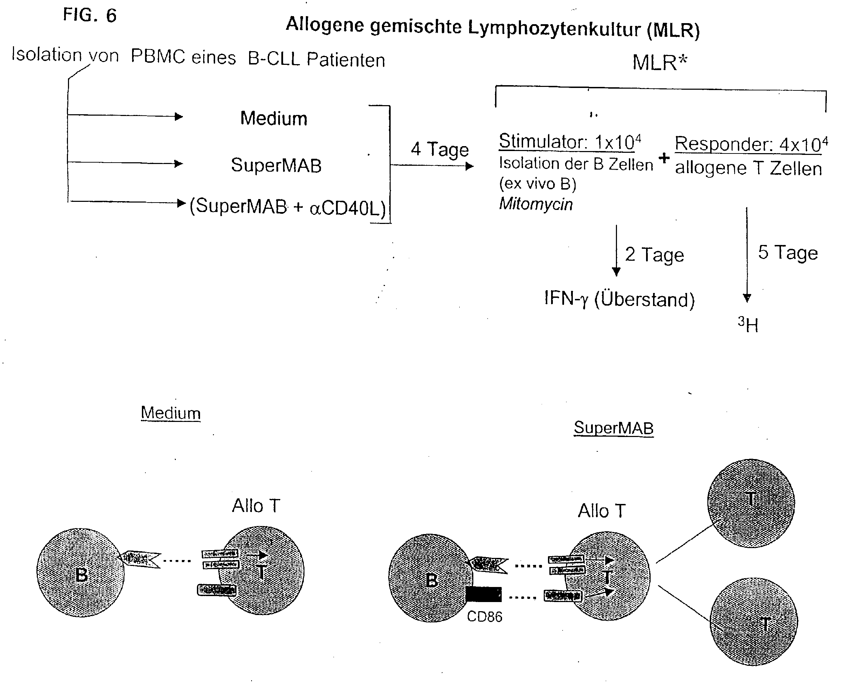

Die entscheidende Frage, ob die Expression kostimulatorischer Liganden auf den B-CLL BZellen auch mit einer verbesserten antigen-präsentierenden Funktion einhergeht, kann nur durch funktionelle Tests geklärt werden: Bei der gemischten Lymphozytenkultur werden T-Zellen einer Person (= Responderzellen) mit sogenannten Stimulator-Zellen zusammen kultiviert. Bei den Stimulator-Zellen handelt es sich um Mitomycin C-behandelte Zellen einer zweiten nicht verwandten Person (= allogener Spender), die im Expresionsmuster der Gene des Haupthistokompatibilitätskomplexes (MHC) von der ersten Person abweicht. Die T-Zellen erkennen die fremden MHC Proteine zusammen mit kostimulierenden Liganden auf der Oberfläche der Stimulatorzellen und werden zur Proliferation angeregt (aufgrund der Mitomycin C-Behandlung können die Stimulatorzellen sich nicht mehr teilen). Nach vier Tagen wird die T-Zell-Proliferation durch den Einbau 3H-Thymidin gemessen (s. Figur 6). Zur Beantwortung der Fragestellung wurden PBMC von B-CLL Patienten isoliert und in Medium oder mit SuperMAB kultiviert (s.o.). Nach vier Tagen wurden die B-CLL B-Zellen aus diesein Kulturen mittels Magnetseparation isoliert, mit Mitomycin C (60 µg/ml) für 1 h bei 37°C inkubiert, und dienten dann als Stimulatorzellen in der sich anschliessenden gemischten Lymphozytenkultur: Hierzu wurden 1 x 10^4 Stimulatorzellen mit 4 x 10^4 T-Zellen eines allogenen Spenders (= Responderzellen) für fünf Tage kultiviert und die Proliferation der T-Zellen durch den Einbau von 3H-Thymidin während der letzten 16h gemessen (s. Figur 6). Figur 4 zeigt, dass nicht mit SuperMAB vorbehandelte B-CLL B-Zellen (Ansätze Medium/ex vivo) eine nur sehr schwache Proliferation der Indikatorzellen hervorrufen können, die durch Zugabe von SuperMAB (Ansatz SuperMAB) deutlich, d.h. mindestens um den Faktor 2, gesteigert werden konnte. Durch Zugabe blockierender monoklonaler Antikörper mit Spezifität für B7.1 und B7.2 (je 5 µg/ml; Ansatz SuperMAB + anti CD80/86) konnte die Proliferation nahezu auf das Niveau der in Medium vorbehandelten Zellen gedrückt werden. Zusammengefasst legen diese Ergebnisse den Schluss nahe, dass SuperMAB die antigen-präsentierende Funktion der B-CLL B-Zellen durch Induktion der kostimulatorischen Proteien B7.1 und B7.2 verbessert.

### Beispiel 5: SuperMAB verbessert die antigenpräsentierende Funktion von B-CLL B-Zellen (autologe gemischte Lymphozytenkultur)

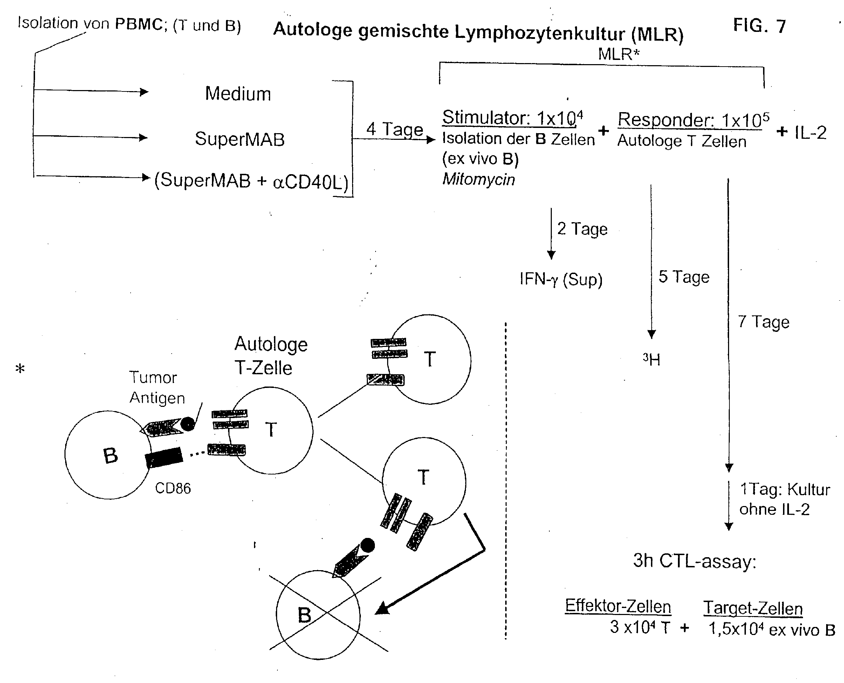

Das Ziel der Immuntherapie bei B-CLL Patienten ist, über die Verbesserung der antigenpräsentierenden Funktion der B-CLL B-Zellen autologe (d.h. körpereigene) tumorspezifische T-Zellen zu aktivieren und diese somit in die Lage zu versetzen, Tumorzellen (d.h. B-CLL B-Zellen) zu eliminieren. Mit Hilfe der sogenannten autologen gemischten Lymphozytenkultur wurde der Frage nachgegangen, ob SuperMAB diesbezügliche Eigenschaften besitzt. Zu diesem Zweck wurden wiederum PBMC eines B-CLL Patienten isoliert und in Medium bzw. in Anwesenheit von SuperMAB kultiviert. Die FACS-Analyse zeigte auch in diesem Experiment ein Induktion von B7.1 und vor allem von B7. 2 in den SuperMAB behandelten B-CLL B-Zellen. Nach drei Tagen wurden die B-CLL B-Zellen aus diesen Kulturen mittels Magnetseparation isoliert, mit Mitomycin C behandelt und sodann als antigenpräsentierende Stimulatorzellen in der sich anschliessenden autologen gemischten Lymphozytenkultur eingesetzt: 1 x 10^4 Stimulatorzellen wurden mit 1 x 10^5 autologen T-Zellen (= Responderzellen) in Anwesenheit des Zytokins Interleukin 2 kultiviert, um die folgende Fragen zu beantworten (s. Figur 7).

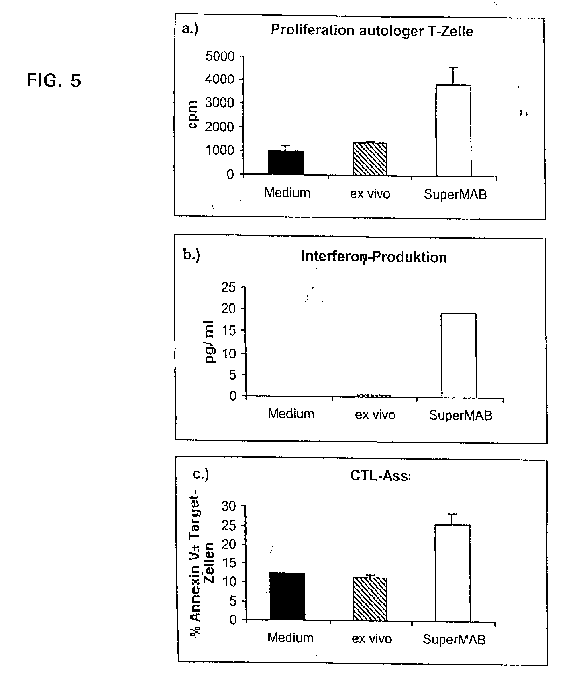

Sind B-CLL B-Zellen aus dem SuperMAB-Ansatz in der Lage, autologe T-Zellen zu aktivieren und zur Proliferation anzutreiben? Als Testsystem diente hier der 3H-Thymidin-Einbau. Ergebnis: im Vergleich zu den Ansätzen Medium/ex vivo konnte durch die Vorbehandlung mit SuperMAB die Proliferation der autologen T-Zellen um den Faktor 4 gesteigert werden (Figur 5a).

Können die so aktivierten T-Zellen das Zytokin Interferon-gamma produzieren? Interferon-gamma wird u.a. von sogenannten Effektor T-Zellen produziert. Zu den Effektor T-Zellen gehören auch die zytotoxischen T-Lymphozyten, die in der Lage sind, Tumorzellen zu eliminieren. Die Konzentration von Interferon-gamma im Zellkulturüberstand wurde mittels ELISA bestimmt. Ergebnis: während in den Ansätzen Medium/ex vivo so gut wie kein Interferon-gamma produziert wurde, konnten die Responder T-Zellen aus dem SuperMAB-Ansatz offensichtlich zur Synthese dieses Zytokins angeregt werden (Figur 5b).

Sind die aktivierten T-Zellen in der Lage, autologe Tumorzellen zu eliminieren? Zur Klärung dieser Frage wurden am Tag 8 der gemischten Lymphozytenkultur 3 x 10^4 Responder T-Zellen (= fragliche Effektor T-Zellen) isoliert und für 3 Stunden mit 1,5 x 10^4 frisch isolierten B-CLL B-Zellen (= Target-Zellen) kultiviert. Danach erfolgte die Inkubation mit dem Protein Annexin V und einem monoklonalen Antikörper mit Spezifität für CD19. Durch Annexin V, an das ein Fluoreszenz-Farbstoff gekoppelt ist, können apoptotische Zellen (das sind Zellen, die aufgrund eines programmierten Zelltodes gestorben sind) durchflusszytometrisch analysiert werden. Eine durch die Behandlung mit SuperMAB erfolgte Induktion potenter tumorspezifischer Effektor T-Zellen und ein daraus resultierendes verstärktes Absterben von Tumorzellen sollte sich daher in einer Erhöhung des Prozentsatzes Annexin V positiver Zellen in der FACS-Analyse niederschlagen. Ergebnis: auch in diesem Test zeigte der SuperMAB-Ansatz das beste Resultat: In den Ansätzen Medium/ex vivo liessen sich ca. 12% der Target-Zellen (= CD19+ Tumorzellen) mit Annexin V anfärben, im SuperMAB-Ansatz dagegen mehr als 25% (Figur 5c).

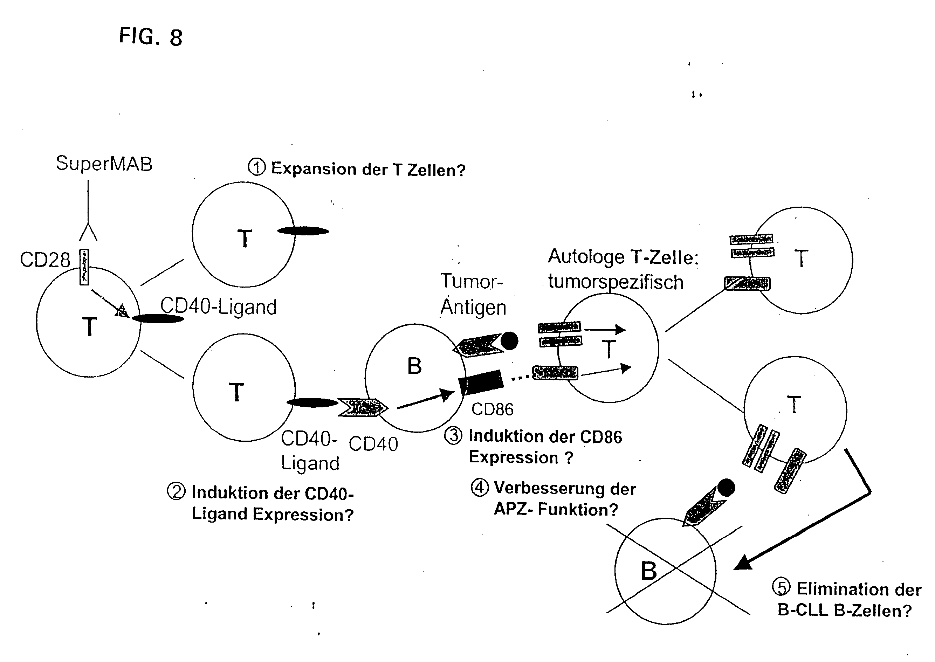

Offensichtlich ist es durch den Einsatz von SuperMAB möglich, tumorspezifische autologe T-Zellen zu aktivieren und eine effiziente Eliminierung der Tumorzellen zu induzieren. Vermutlich ist dies auf eine Induktion kostimulatorischer Proteine auf der Oberfläche der antigenpräsentierenden B-CLL B-Zellen zurückzuführen sind. Der mögliche Mechanismus ist in der Figur 8 schematisch dargestellt.

### Beispiel 6: Herstellung eines erfindungsgemäßen monoklonalen Antikörpers TGN1412.

Folgend wird die Herstellung eines erfindungsgemäßen monoklonalen Antikörpers TGN1412 beschrieben, welcher aus den in den Figuren 9a und 9b angegebenen Aminosäurensequenzen der leichten und der schweren Kette besteht.

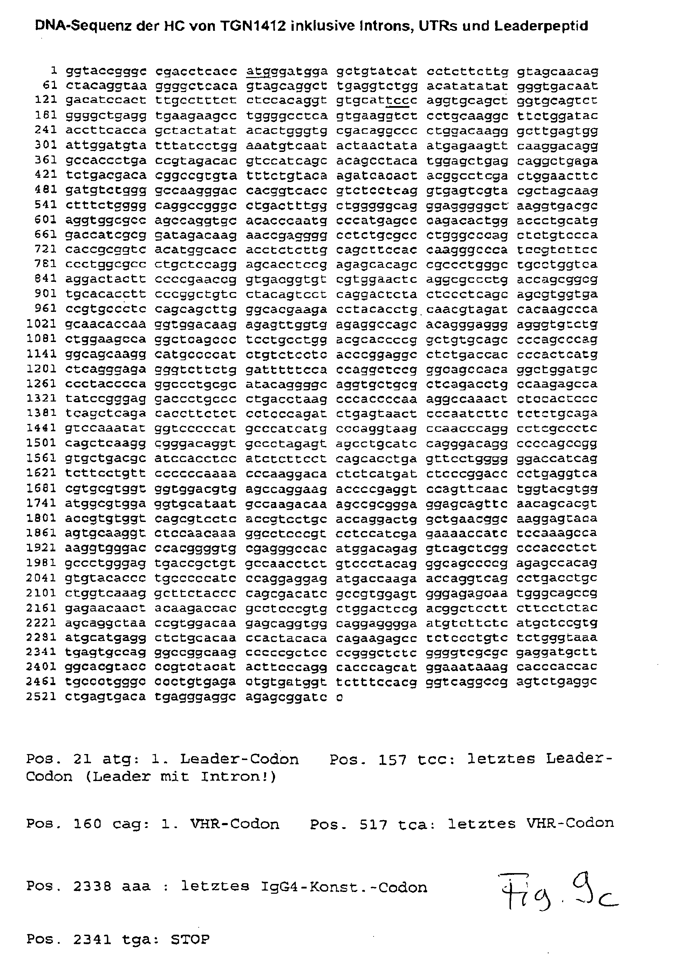

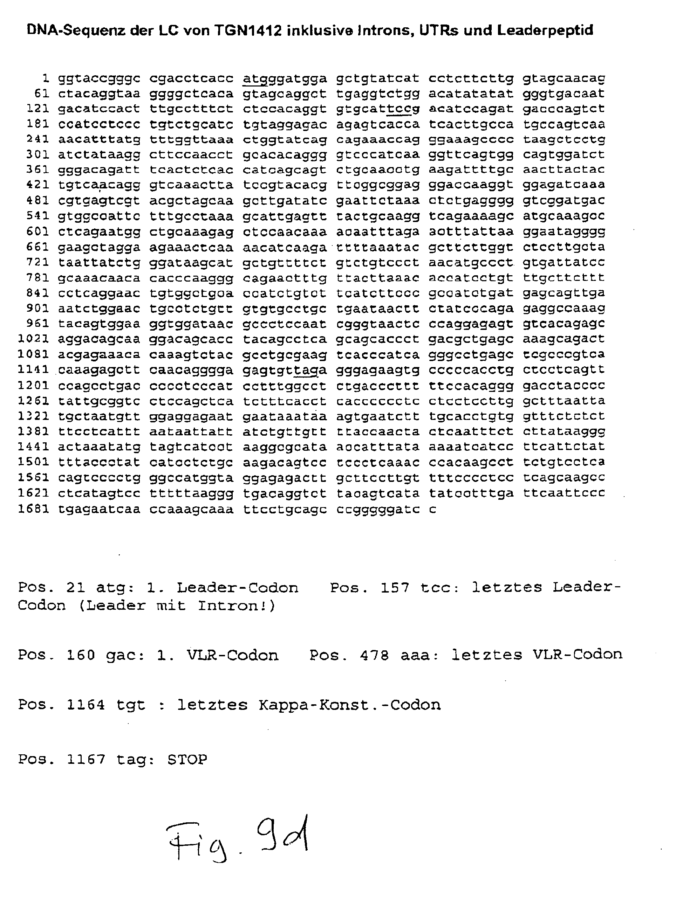

Die in der Figuren 9c und 9d angegebenen DNA Moleküle wurden in fachüblicher Weise synthetisiert. Die erhaltenen cDNAs, codierend jeweils für die schwere und die leichte Kette des monoklonalen Antikörpers wie angegeben, wurden in Plasmide pLNOH und pLNOK insertiert. Zur Verfahrensweise im Einzelnen, den Plasmiden und den Schnittstellen wird auf die Literaturstelle Norderhaug et al., Journal of Immunological Methods, 204:77-87 (1997) verweisen.

Mit den jeweiligen Plasmiden wurden E. coli Zellen transformiert. Die transformierten E. coli Zellen wurden vermehr und die jeweiligen Plasmide wurden in größeren Maßstab hieraus gewonnen.

Mit den erhaltenen jeweiligen Plasmiden, enthaltend die DNA Information für die komplette schwere oder leichte Kette, wurden COS7-Zellen transient und CHO- sowie NSO-Zellen stabil cotransfiziert. Die Cotransfektion erfolgte in üblicher Weise mit Lipofectamine (Invitrogen) oder Effectene (QIAGEN) nach den jeweiligen Herstellervorschriften.

Die erhaltenen transfizierten Zellen wurden in Standard-Kulturmedium in CO₂-Inkubatoren (5% CO₂) bei 37°C in T-flasks und Rollerflaschen kultiviert. Stabil transfizierte Zellen, welche den intakten Antikörper exprimieren, wurden mittels G418- und Hygromycin B-Selektion selektiert. Stabile Transformanten wurden dann subkloniert und weiter kultiviert.

Zellkulturüberstände mit sekretierten Antikörpern wurden analysiert mittels Aktivitätsassays, Bindungsassays (FACS-Analyse), SDS-PAGE und western blotting.

Zur Aufreinigung wurden Zellkulturüberstände über eine Protein A oder Protein G Säule geleitet, um die gewünschten Antikörper spezifisch zu binden. Gebundene Antikörper wurden mit acidischem Puffer (0,1 M Glycin, pH 3,0 - 3,4) eluiert und anschließend mit 1 M Tris (pH 8,0) neutralisiert. Die so gereinigten Antikörper wurden gegen PBS Puffer dialysiert und nach Erfordernissen ggf. mittels Membranfiltration aufkonzentriert. Die erhaltenen Antikörper wurden dann für Proliferationsassays, Aktivitätsassays, Bindungsassays (FACS Analyse auf Bindung an CD28 positive Zellen), ELISA, SDS-PAGE und western blotting verwendet.

## Patentansprüche

1. Verwendung eines superagonistischen monoklonalen Antikörpers (mAb), welcher für auf T-Zellen exprimiertes CD28 spezifisch ist, zur Herstellung einer pharmazeutischen Zusammensetzung zur Behandlung von der chronisch lymphozytäre Leukämie des B-Zell-Typs (B-CLL).

2. Verwendung nach Anspruch 1, wobei der mAb herstellbar ist, indem ein nichtmenschliches Säugetier mit CD28 oder einer Teilsequenz hieraus immunisiert wird, wobei aus dem nichtmenschlichen Säugetier Zellen entnommen und aus den Zellen Hybridomzellen hergestellt werden, und wobei die so erhaltenen Hybridomzellen mit der Maßgabe selektiert werden, dass in deren Kulturüberstand mAb enthalten sind, die an CD28 superagonistisch binden.

3. Verwendung nach einem der Ansprüche 1 oder 2,
wobei der mAb erhältlich ist aus Hybridomzellen, wie hinterlegt unter den DSM Nummern DSM ACC2531 (mAb: 9D7 bzw. 9D7G3H11) oder DSM ACC2530 (mAb: 5.11A bzw. 5.11A1C2H3), oder
wobei der Antikörper in der schweren Kette eine Aminosäuresequenz für die variable Region gemäß Figur 9a (VHC) enthält und in der leichten Kette eine Aminosäuresequenz für die variable Region gemäß Fig. 9b (VLC) enthält, wobei schwere und leichte Kette optional zusätzlich die jeweilig in den Figuren 9a und 9b angegebenen Aminosäuresequenzen für die konstante Region der schweren Kette (CHC) und die konstante Region der leichten Kette (CLC) enthalten (in den dort angegebenen alternativen Varianten), wobei die schwere Kette und leichte Kette zusätzlich optional die jeweilig in den Figuren 9a und 9b angegebenen Aminosäuresequenzen für das Leaderpeptid enthalten, wobei die schwere Kette und die leichte Kette vorzugsweise jeweils aus den in den Figuren 9a und 9b dargestellten Volllängensequenzen bestehen (in den alternativen Varianten des C-Terminus, wie in den Figuren 9a und 9b angegeben).

4. Verwendung nach Anspruch 1, wobei die pharmazeutische Zusammensetzung galenisch zur i.v. Injektion, hergerichtet ist.

5. Verfahren zur Reduktion von B-CLL B-Zellen und Expansion von T-Zellen in einer normale T-Zellen, B-Zellen sowie B-CLL B-Zellen enthaltenden Flüssigkeit, die sich außerhalb eines Körpers befindet,
wobei durch Wechselwirkung der in der Flüssigkeit vorliegenden normalen T-Zellen mit einem für einen natürlicherweise kostimulatorischen Rezeptor auf ruhenden T-Zellen, nämlich CD28, spezifischen superagonistischen mAb die normalen T-Zellen aktiviert bzw. expandiert werden,
wobei durch Wechselwirkung der in der Flüssigkeit vorliegenden B-CLL T-Zellen mit dem CD28-spezifischen superagonistischen mAb zur Expression eines CD40 Liganden aktiviert und ggf. expandiert werden,
wobei die aktivierten bzw. expandierten CD40 Liganden tragenden B-CLL T-Zellen in CD40 tragenden B-CLL B-Zellen die Expression von CD86 und/oder CD80 induzieren,
wobei autologe tumorspezifische T-Zellen durch die CD86 und/oder CD80 tragenden B-CLL B-Zellen costimulatorisch aktiviert bzw. expandiert werden, und
wobei die aktivierten tumorspezifischen T-Zellen die B-CLL B-Zellen eliminieren.

## Claims

1. A use of a superagonistic monoclonal antibody (mAb), which is specific for CD28 expressed on T cells, for producing a pharmaceutical composition for the treatment of the chronic lymphocytic leukemia of the B-cell type (B-CLL).

2. The use according to claim 1, wherein the mAb can be produced by that a non-human mammal is immunized with CD28 or a partial sequence thereof, wherein cells are taken from the non-human mammal, and hybridoma cells are produced from the cells, and wherein the thus obtained hybridoma cells are selected such that in their culture supernatant there are contained mAb's which superagonistically bind to CD28.

3. The use according to one of claims 1 or 2,
wherein the mAb can be obtained from hybridoma cells, as filed under the DSM numbers DSM ACC2531 (mAb: 9D7 or 9D7G3H11) or DSM ACC2530 (mAb: 5.11A or 5.11A1C2H3), or
wherein the antibody in the heavy chain contains an amino acid sequence for the variable region according to Figure 9a (VHC) and in the light chain an amino acid sequence for the variable region according to Figure 9b (VLC), wherein the heavy and light chains optionally in addition contain the amino sequences according to Figures 9a and 9b, respectively, for the constant region of the heavy chain (CHC) and the constant region of the light chain (CLC) (in the alternative variants shown there), wherein the heavy and light chains optionally in addition contain the amino acid sequences for the leader peptide shown in Figures 9a and 9b, wherein the heavy and light chains preferably consist of the full-length sequences shown in Figures 9a and 9b, respectively (in the alternative variants of the C-terminus, as shown in Figures 9a and 9b).

4. The use according to claim 1, wherein the pharmaceutical composition is galenically prepared for IV injection.

5. A method for reducing B-CLL B cells and expanding T cells in a liquid containing normal T cells, B cells and B-CLL B cells, said liquid being outside of a body,
wherein by interaction of the normal T cells present in the liquid with a superagonistic mAb specific for a naturally costimulatory receptor on resting T cells, namely CD28, the normal T cells are activated or expanded,
wherein by interaction of the B-CLL T cells present in the liquid with the superagonistic mAb specific for CD28, the B-CLL T cells are activated or expanded for the expression of a CD40 ligand,
wherein the activated or expanded B-CLL T cells carrying CD40 ligands induce the expression of CD86 and/or CD80 in B-CLL B cells carrying CD40,
wherein autologous tumor-specific T cells are costimulatorily activated or expanded by the B-CLL B cells carrying CD86 and/or CD80, and
wherein the activated tumor-specific T cells eliminate the B-CLL B cells.

## Revendications

1. Utilisation d'un anticorps monoclonal (acm) super-agonistique, qui est spécifique de CD28 exprimé sur des cellules T, pour la production d'une composition pharmaceutique pour le traitement de la leucémie chronique lymphocytaire du type à cellules B (B-CLL).

2. Utilisation selon la revendication 1, dans laquelle l'acm peut être produit par ce qu'un mammifère non humain est immunisé avec CD28 ou une séquence partielle de celui-ci, dans laquelle des cellules sont prélevées sur le mammifère non humain, et des cellules d'hybridome sont produites à partir des cellules, et dans laquelle les cellules d'hybridome ainsi obtenues sont sélectionnées de telle façon, que dans le surnageant de culture de celles-ci des acm's soient contenus, qui se lient de manière super-agonistique à CD28.

3. Utilisation selon une des revendications 1 ou 2,
dans laquelle l'acm peut être obtenu à partir de cellules d'hybridome, comme déposées sous les numéros DSM ACC2531 (acm: 9D7 ou 9D7G3Hll, respectivement) ou DSM ACC2530 (acm: 5.11A ou 5.11A1C2H3, respectivement), ou
dans laquelle l'anticorps contient dans la chaîne lourde une séquence d'acides aminés pour la région variable selon la figure 9a (VHC) et dans la chaîne légère une séquence d'acides aminés pour la région variable selon la figure 9b (VLC), dans laquelle la chaîne lourde et la chaîne légère optionnellement en addition contiennent les séquences d'acides aminés respectivement montrées dans les figures 9a et 9b pour la région constante de la chaîne lourde (CHC) et la région constante de la chaîne légère (CLC) (dans les variantes alternatives montrées), dans laquelle la chaîne lourde et la chaîne légère optionnellement en addition contiennent les séquences d'acides aminés respectivement montrées dans les figures 9a et 9b pour le peptide leader, dans laquelle la chaîne lourde et la chaîne légère de préférence respectivement consistent en les séquences complètes montrées dans les figures 9a et 9b (dans les variantes alternatives de la terminaison C, comme montré dans les figures 9a et 9b).

4. Utilisation selon la revendication 1, dans laquelle la composition pharmaceutique est préparée galéniquement pour l'injection i.v.

5. Procédé de réduction de cellules B-CLL B et d'expansion de cellules T dans un liquide contenant des cellules T normales, cellules B et cellules B-CLL B, ce liquide se trouvant à l'extérieur d'un corps,
dans laquelle par interaction des cellules T normales présentes dans le liquide avec un acm super-agonistique spécifique d'un récepteur de co-stimulation naturelle sur des cellules T quiescentes, c'est-à-dire CD28, les cellules T normales sont activées ou dilatées,
dans laquelle par interaction des cellules B-CLL T présentes dans le liquide avec l'acm super-agonistique spécifique de CD28, les cellules B-CLL T sont activées ou, le cas échéant, dilatées pour l'expression d'un ligand CD40,
dans laquelle les cellules B-CLL T activées ou dilatées portant des ligands CD40 induisent l'expression de CD86 et/ou CD80 dans des cellules B-CLL B portant CD40,
dans laquelle des cellules T autologues spécifiques de tumeurs sont activées ou dilatées de manière costimulante par les cellules B-CLL B portant CD86 et/ou CD80, et
dans laquelle les cellules T activées spécifiques de tumeurs éliminent les cellules B-CLL B.


### SEQUENCE LISTING

<110> TeGenero Ah
<160> 19
<170> Patent In version 3.1
<210> 1
   <211> 6
   <212> PRT
   <213> Artificial
<400> 1
   Val Tyr Ser Lys Thr Gly
   1               5
<210> 2
   <211> 4
   <212> PRT
   <213> Artificial
<400> 2
   Tyr Ser Lys Thr
   1
<210> 3
   <211> 5
   <212> PRT
   <213> Artificial
<400> 3
   Tyr Ser Lys Thr Gly
   1               5
<210> 4
   <211> 5
   <212> PRT
   <213> Artificial
<400> 4
   Val Tyr Ser Lys Thr
   1 5
<210> 5
   <211> 6
   <212> PRT
   <213> Artificial
<400> 5
   Tyr Ser Lys Thr Gly Phe
   1 5
<210> 6
   <211> 7
   <212> PRT
   <213> Artificial
<400> 6
   Val Tyr Ser Lys Thr Gly Phe
   1 5
<210> 7
   <211> 5
   <212> PRT
   <213> Artificial
<400> 7
   Ser Lys Thr Gly Phe
   1 5
<210> 8
   <211> 8
   <212> PRT
   <213> Artificial
<400> 8
   Gly Asn Tyr Ser Gln Gln Leu Gln
   1 5
<210> 9
   <211> 321
   <212> DNA
   <213> Artificial
<400> 9
   gatatccaga cgacacagac tacatcctcc cgttctgcct ctctgggaga cagagtcacc 60
   atcagttgca gggcaggtca ggacattagt aattatttaa actggtatca gcagaaacca 120
   gatggaactg ttaagctcct gatctactac acatcaagat tacactcagg agtcccatca 180
   aggttcagtg gcagtgggtc tggaacagat tattctctca ccattagcaa cctggagcaa 240
   gaagatattg ccacttactt ttgccaacag ggtcatacgc ttccgtggac gttcggtgga 300
   ggcaccaagc tggaaatcaa a 321
<210> 10
   <211> 107
   <212> PRT
   <213> Artificial
<400> 10
   Asp Ile Gln Thr Thr Gln Thr Thr Ser Ser Leu Ser Ala Ser Leu Gly
   1 5 10 15
   Asp Arg Val Thr Ile Ser Cys Arg Ala Gly Gln Asp Ile Ser Asn Tyr
   20 25 30
   Leu Asn Trp Tyr Gln Gln Lys Pro Asp Gly Thr Val Lys Leu Leu Ile
   35 40 45
   Tyr Tyr Thr Ser Arg Leu His Ser Gly Val Pro Ser Arg Phe Ser Gly
   50 55 60
   Ser Gly Ser Gly Thr Asp Tyr Ser Leu Thr Ile Ser Asn Leu Glu Gln
   65 70 75 80
   Glu Asp Ile Ala Thr Tyr Phe Cys Gln Gln Gly His Thr Leu Pro Trp
   85 90 95
   Thr Phe Gly Gly Gly Thr Lys Leu Glu Ile Lys
   100 105
<210> 11
   <211> 363
   <212> DNA
   <213> Artificial
<400> 11
   gatgtgcagc ttcaggagtc gggacctggc ctggtgaaac cttctcagtc tctgtccctc 60
   acctgcactg tcactggcta ctcaatcacc agtgattatg cctggaactg gatccggcag 120
   tttccaggaa acaaactgga gtggatgggc tacataagat acagtggtag tactagctac    180
   aatccatctc tcaaaagtcg aatctctatc actcgagaca catccaagaa ccagttcttc    240
   ctgcagttga attctgtgac tactgaggac acagccacat attactgtgc aagagattgg    300
   ccgcgaccga gctactggta cttcgatgtc tggggcgcag ggaccacggt caccgtctcc    360
   tca                                                                  363
<210> 12
   <211> 121
   <212> PRT
   <213> Artificial
<400> 12
   Asp Val Gln Leu Gln Glu Ser Gly Pro Gly Leu Val Lys Pro Ser Gln
   1               5                   10                  15
   Ser Leu Ser Leu Thr Cys Thr Val Thr Gly Tyr Ser Ile Thr Ser Asp
               20                  25                  30
   Tyr Ala Trp Asn Trp Ile Arg Gln Phe Pro Gly Asn Lys Leu Glu Trp
           35                  40                  45
   Met Gly Tyr Ile Arg Tyr Ser Gly Ser Thr Ser Tyr Asn Pro Ser Leu
       50                  55                  60
   Lys Ser Arg Ile Ser Ile Thr Arg Asp Thr Ser Lys Asn Gln Phe Phe
   65                  70                  75                  80
   Leu Gln Leu Asn Ser Val Thr Thr Glu Asp Thr Ala Thr Tyr Tyr Cys
                   85                  90                  95
   Ala Arg Asp Trp Pro Arg Pro Ser Tyr Trp Tyr Phe Asp Val Trp Gly
               100                 105                 110
   Ala Gly Thr Thr Val Thr Val Ser Ser
           115                 120
<210> 13
   <211> 360
   <212> DNA
   <213> Artificial
<400> 13
   caggtccaac tgcagcagtc cggacctgag ctggtgaagc cggggacttc agtgaggatt     60
   tcctgcgagg cttctggcta caccttcaca agctactata tacactgggt gaaacagagg    120
   cctggacagg gacttgagtg gattggatgt atttatcctg gaaatgtcaa tactaactat    180
   aatgagaagt tcaaggacaa ggccacactg attgtagaca catcctccaa cactgcctac    240
   atgcagctca gcagaatgac ctctgaggac tctgcggtct atttctgtac aagatcacac    300
   tacggcctcg actggaactt cgatgtctgg ggcgcaggga ccacggtcac cgtctcctca    360
<210> 14
   <211> 120
   <212> PRT
   <213> Artificial
<400> 14
   Gln Val Gln Leu Gln Gln Ser Gly Pro Glu Leu Val Lys Pro Gly Thr
   1               5                   10                  15
   Ser Val Arg Ile Ser Cys Glu Ala Ser Gly Tyr Thr Phe Thr Ser Tyr
               20                  25                  30
   Tyr Ile His Trp Val Lys Gln Arg Pro Gly Gln Gly Leu Glu Trp Ile
           35                  40                  45
   Gly Cys Ile Tyr Pro Gly Asn Val Asn Thr Asn Tyr Asn Glu Lys Phe
       50                  55                  60
   Lys Asp Lys Ala Thr Leu Ile Val Asp Thr Ser Ser Asn Thr Ala Tyr
   65                  70                  75                  80
   Met Gln Leu Ser Arg Met Thr Ser Glu Asp Ser Ala Val Tyr Phe Cys
                   85                  90                  95
   Thr Arg Ser His Tyr Gly Leu Asp Trp Asn Phe Asp Val Trp Gly Ala
               100                 105                 110
   Gly Thr Thr Val Thr Val Ser Ser
           115                 120
<210> 15
   <211> 321
   <212> DNA
   <213> Artificial
<400> 15
   gacatccaga tgaaccagtc tccatccagt ctgtctgcat cccttggaga cacaattacc      60
   atcacttgcc atgccagtca aaacatttat gtttggttaa actggtacca gcagaaacca     120
   ggaaatattc ctaaactctt gatctataag gcttccaacc tgcacacagg cgtcccatca     180
   aggtttagtg gcagtggatc tggaacaggc ttcacattaa ccatcagcag cctgcagcct     240
   gaagacattg ccacttacta ctgtcaacag ggtcaaactt atccgtacac gttcggaggg     300
   gggaccaagc tggaaataaa a                                               321
<210> 16
   <211> 107
   <212> PRT
   <213> Artificial
<400> 16
   Asp Ile Gln Met Asn Gln Ser Pro Ser Ser Leu Ser Ala Ser Leu Gly
   1               5                   10                  15
   Asp Thr Ile Thr Ile Thr Cys His Ala Ser Gln Asn Ile Tyr Val Trp
               20                  25                  30
   Leu Asn Trp Tyr Gln Gln Lys Pro Gly Asn Ile Pro Lys Leu Leu Ile
           35                  40                  45
   Tyr Lys Ala Ser Asn Leu His Thr Gly Val Pro Ser Arg Phe Ser Gly
       50                  55                  60
   Ser Gly Ser Gly Thr Gly Phe Thr Leu Thr Ile Ser Ser Leu Gln Pro
   65                  70                  75                  80
   Glu Asp Ile Ala Thr Tyr Tyr Cys Gln Gln Gly Gln Thr Tyr Pro Tyr
                   85                  90                  95
   Thr Phe Gly Gly Gly Thr Lys Leu Glu Ile Lys
               100                 105
<210> 17
   <211> 15
   <212> PRT
   <213> Artificial
<400> 17
   Gly Asn Tyr Ser Gln Gln Leu Gln Val Tyr Ser Lys Thr Gly Phe
   1               5                   10                  15
<210> 18
   <211> 107
   <212> PRT
   <213> Artificial
<400> 18
   Asp Ile Gln Met Thr Gln Ser Pro Ser Ser Leu Ser Ala Ser Val Gly
   1               5                   10                  15
   Asp Arg Val Thr Ile Thr Cys His Ala Ser Gln Asn Ile Tyr Val Trp
               20                  25                  30
   Leu Asn Trp Tyr Gln Gln Lys Pro Gly Lys Ala Pro Lys Leu Leu Ile
           35                  40                  45
   Tyr Lys Ala Ser Asn Leu His Thr Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly Gln Thr Tyr Pro Tyr
                   85                  90                  95
   Thr Phe Gly Gly Gly Thr Lys Val Glu Ile Lys
               100                 105
<210> 19
   <211> 120
   <212> PRT
   <213> Artificial
<400> 19
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
   20 25 30
   Tyr Ile His Trp Val Arg Gln Ala Pro Gly Gln Gly Leu Glu Trp Ile
   35 40 45
   Gly Cys Ile Tyr Pro Gly Asn Val Asn Thr Asn Tyr Asn Glu Lys Phe
   50 55 60
   Lys Asp Arg Ala Thr Leu Thr Val Asp Thr Ser Ile Ser Thr Ala Tyr
   65 70 75 80
   Met Glu Leu Ser Arg Leu Arg Ser Asp Asp Thr Ala Val Tyr Phe Cys
   85 90 95
   Thr Arg Ser His Tyr Gly Leu Asp Trp Asn Phe Asp Val Trp Gly Gln
   100 105 110
   Gly Thr Thr Val Thr Val Ser Ser
   115 120